# EUROPÄISCHE PATENTSCHRIFT

(11) **EP 2 952 172 B1**
(45) Veröffentlichungstag und Bekanntmachung des Hinweises auf die Patenterteilung: **28.11.2018**
(21) Anmeldenummer: 15170488.9
(22) Anmeldetag: 03.06.2015
(51) Int. Cl.: A61K 6/00

(54) **HAFTMITTEL FÜR ZAHNPROTHESEN UND VERFAHREN ZU SEINER HERSTELLUNG**
BONDING AGENT FOR DENTAL PROSTHESES AND METHOD OF PREPARING SAME
PRODUIT ADHÉSIF POUR PROTHÈSES DENTAIRES ET SON PROCÉDÉ DE FABRICATION

(30) Priorität: 04.06.2014 AT 503902014
(43) Veröffentlichungstag der Anmeldung: 09.12.2015
(73) Patentinhaber: Magister Hoeveler & Co Gesellschaft m.b.H., 1230 Wien (AT)
(72) Erfinder: von Nagy, Robert, 3021 Pressbaum (AT)
(74) Vertreter: Patentanwälte Barger, Piso & Partner

(56) Entgegenhaltungen:
- EP-A1- 0 407 681
- US-A- 3 811 981
- US-A1- 2003 027 887
- US-A1- 2008 233 058

## Beschreibung

Die Erfindung betrifft ein Haftmittel für Zahnprothesen im Ober- und Unterkieferbereich in Form einer pastösen, leicht verstreichbaren, Cellulosederivate und/oder Hydrokolloide und/oder mikrokristallines Carboxymethylcellulose(CMC)-Granulat, Glycerintriacetat, Silica und Aqua purificata enthaltenden Masse entsprechend dem Oberbegriff des Anspruches 1 und der US 2003/0027887 A1; sowie ein Verfahren zur Herstellung des Haftmittels.

Die US 2003/0027887 A1 offenbart ein Haftmittel für Zahnprothesen in Form einer pastösen Masse, die ein Terpolymer, Carboxymethylcellulose und Silica enthält, Aqua purificata ist als bevorzugtes Lösungsmittel genannt und Zink ist zwingend vorgeschrieben und zum Erhalt der angestrebten Eigenschaften auch notwendig. Diese Anwesenheit ist medizinisch bedenklich und in vielen Jurisdiktionen unzulässig.

Aus der EP 407 681 A1 ist eine Hafteinlage mit einem Faservlies bekannt, das mit einem Haftstoff enthaltend Carboxymethylcellulose und oder andere Hydrocolloide, Polyvinylacetat und ein wasserunlösliches Lösungsmittel, wie Glycerin-Triacetat, getränkt ist.

Aus der US 3,811,981 ist ein Verfahren zum Kleben von Papier bekannt, bei dem der Klebstoff ein Terpolymer aus 65 Gew.-% Vinylacetat, 30 Gew.-% Vinyllaurat und 5 Gew.-% Crotonsäure enthält.

Aus dem Stand der Technik, beispielsweise der EP 229 010 B1 oder US 4,804,412 ist ein Haftmittel für Zahnprothesen bekannt, das eine streichfähige Masse umfasst, die aus einer Mischung eines Alginats und eines Polyvinylacetats mit Carboxymethylcellulose, einem Methylcellulose-Emulgator und einem Neutralölweichmacher sowie einem alkoholischen Lösungsmittel, wie 85%iger Ethylalkohol besteht.

In der AT 509 474 B1 ist weiters ein Haftmittel für Zahnprothesen im Ober- und Unterkieferbereich in Form einer pastösen, leicht verstreichbaren Masse geoffenbart, die unter anderem Polyvinylacetate, Hydrokolloide, mikrokristallines CMC-Granulat, Glycerintriacetat und ein alkoholisches Lösungsmittel enthält.

Den vorbekannten Haftmitteln ist zumeist gemeinsam, dass jeweils alkoholische Lösungsmittel, wie Ethylalkohol, darin verwendet werden, welcher Umstand jedoch mit Nachteilen verbunden ist. Zum einen führt die leichte Verdunstbarkeit des alkoholischen Lösungsmittels zu einem Austrocknen des Haftmittels, wodurch die Verstreichbarkeit bzw. eine zweckbestimmte Anwendbarkeit des Haftmittels erheblich beeinträchtigt wird. Zum anderen gilt es für viele Verbraucher von derartigen Produkten die Anwendung alkoholischer Lösungsmittel zu vermeiden.

Des Weiteren ist aus dem Stand der Technik die DE 19704293 A1 bekannt, die ein Haftmittel offenbart, das als Wirkstoffe Copolymerisate enthält, die die Haftkraft der Zahnprothesenhaftmittel weiter erhöhen, einen in Zahnprothesenhaftmitteln üblichen Träger enthält und bevorzugt lösungsmittelfrei hergestellt werden kann. Das genannte Haftmittel umfasst u.a. Alkylvinyle, Crotonsäure, Cellulosederivate und Glycerin.
Die WO 006072 A offenbart ein Haftmittel, das Terpolymere enthält, umfassend Alkylvinylpolymere sowie Cellulosederivate und Glycerin, Silconderivate und Maleinsäurederivate.

Die EP 040768 A1 beschreibt eine Hafteinlage für Zahnprothesen, bestehend aus einem haftstoffdurchsetzten Faservlies mit Haftstoff aus einer zähflüssigen Mischung von Alginat und/oder Carboxymethylcellulose und andere Hydrokolloide, Polyvinylacetat und einem wasserunlöslichen Lösungsmittel, wie Glycerin-Triacetat.

Die vorgenannten Haftmittel haben eine hohe Haftkraft, sind wasserunlöslich und mit einem alkoholfreien Lösungsmittel herstellbar. Sie sind jedoch alle mit dem Nachteil verbunden, dass die mit den Haftmitteln versehenen Zahnprothesen sich schwierig reinigen lassen, zumal diese Haftmittel offensichtlich zufolge ihrer hohen nachhaltigen Wasserunlöslichkeit fest mit der Zahnprothese verklebt bleibt.

Aufgabe der Erfindung ist es nun, ein weiteres Haftmittel für Zahnprothesen im Ober- und Unterkieferbereich in Form einer pastösen, leicht verstreichbaren Masse zu schaffen, die ohne bedenkliche Inhalts Stoffe, alkoholfrei und dabei über einen längeren Zeitraum hin wasserunlöslich ist, wobei auch die oben dargelegten Nachteile ausgeschaltet sind.

Diese Aufgabe wird erfindungsgemäß mit einem Haftmittel der oben angegebenen Art dadurch gelöst, dass die Masse ein Terpolymer mit der Monomerzusammensetzung von 57 Gew.-% Vinylacetat, 40 Gew.-% Vinyllaurat und 3 Gew.-% Crotonsäure enthält. Ein solches Terpolymer ist unter der Produktbezeichnung CAPIVA®DA kommerziell von der Wacker Chemie AG erhältlich. Es wird nachfolgend einfach als Terpolymer bezeichnet.

Vorteilhafterweise ist das Haftmittel durch diese Terpolymerlösung zumindest über einen längeren Zeitraum hin wasserunlöslich und dabei alkoholfrei. Dadurch dass die weitgehende Wasserunlöslichkeit des Haftmittels nach einer gewissen Zeit schwächer wird, ist auch an der Zahnprothese anhaftendes restliches Haftmittel zu Reinigungszwecken der Zahnprothese leichter entfernbar. Dieses überraschend gefundene vorteilhafte Verhalten des Haftmittels ist der ganz speziellen Formulierung des Haftmittels zuzuschreiben. Überdies gibt es kein Austrocknen des Haftmittels in der Tube, wie dies bei anderen bekannten Haftmitteln auftritt, die wasserunlöslich sind und einen Ethanolanteil aufweisen. Außerdem verspüren Patienten und Anwender des erfindungsgemäßen Haftmittels, die sensibel auf Alkohol reagieren, durch den Wegfall des Ethanols kein Brennen mehr im Mund. Das erfindungsgemäße Haftmittel ohne Alkoholkomponente kann nunmehr auch ohne Probleme in aller Welt vertrieben werden.
Ferner enthält erfindungsgemäß die Masse des Haftmittels zusätzlich Xanthan, mittels welchem wirksam eine weitgehende Unterbindung einer Entmischung/Synärese des Haftmittels unterstützt wird.
Nach einem weiteren Merkmal der Erfindung umfasst das Haftmittel eine Masse, die 20 - 24 Gew.-Teile Terpolymer, 22 - 29 Gew.-Teile Glycerintriacetat, 2 - 6 Gew.-Teile Paraffinum liquidum, 32 - 44 Gew.-Teile Cellulosederivate und/oder Hydrokolloide und/oder mikrokristallines CMC-Granulat, 1 - 3 Gew.-Teile Silica und 2 - 4 Gew.-Teile Aqua purificata aufweist. Eine derartige Haftmittelmasse weist eine ausgezeichnete Geschmeidigkeit bei hoher und lang andauernder Klebkraft auf und lässt sich leicht auf die Zahnprothese aufbringen, ohne dass in der Folge eine Irritation des Gaumens oder Zahnfleischs auftritt. Außerdem ist diese Haftmittelmasse weitgehend unabhängig vom Speichelfluss. Dabei sind die Bestandteile der Haftmittelmasse Cellulosederivate, Hydrokolloide und mikrokristallines CMC-Granulat gewünschtenfalls beliebig gegeneinander austauschbar.
Das erfindungsgemäße Haftmittel ist insbesondere dadurch gekennzeichnet, dass die Masse 22 Gew.-Teile Terpolymer, 25 Gew.-Teile Glycerintriacetat, 3 Gew.-Teile Aqua purificata, 4 Gew.-Teile Paraffinum liquidum, 3 Gew.-Teile Silica und 43 Gew.-Teile mikrokristallines CMC-Granulat und/oder Hydrokolloide und/oder Cellulosederivate aufweist. Nach einem weiteren Merkmal der Erfindung weist die Masse des Haftmittels 24 Gew.-Teile Terpolymer, 27 Gew.-Teile Glycerintriacetat, 4 Gew.-Teile Aqua purificata, 2 Gew.-Teile Paraffinum liquidum, 6 Gew.-Teile Silica, 36 Gew.-Teile mikrokristallines CMC-Granulat und/oder Hydroklloide und/oder Cellulosederivat und 1 Gew.-Teil Xanthan auf. Die Erfindung betrifft auch ein Verfahren zur Herstellung des Haftmittels für Zahnprothesen im Ober- und Unterkieferbereich das dadurch gekennzeichnet ist, dass eine erste Mischung durch Vermischen von Terpolymer mit einer Monomerzusammensetzung 57 Gew.-% Vinylacetat, 40 Gew.-% Vinyllaurat und 3 Gew.-% Crotonsäure, Glycerintriacetat und Aqua purificata zu einer zähflüssigen durchscheinenden Masse zubereitet wird, dass dann in die so hergestellte Masse in einem Mischkneter Paraffinum liquidum eingerührt wird, dass danach ein vorgemischtes Gemenge von Silica und mikrokristallinem CMC-Granulat kontinuierlich unter intensivem Rühren der Mischung zugesetzt wird, worauf nach einem Mischgang die Mischdrehrichtung umgekehrt, daran anschließend das Mischgut umgepumpt und schließlich auf eine Temperatur von ca. 43-45° C gebracht wird.

Diese Verfahrenscharakteristik ist auch für die vorteilhaften oben angegebenen Eigenschaften des Haftmittels verantwortlich.

Das Verfahren ist ferner dadurch gekennzeichnet, dass dem vorgemischten Gemenge von Silica und mikrokristallinem CMC-Granulat Xanthan zugesetzt wird.

Der Vorteil des Verfahrens gemäß der Erfindung ergibt sich weiters auch daraus, dass bei dem erfindungsgemäßen Haftmittel kein Alkohol verwendet wird, so dass auch bei den Fertigungsanlagen für das Haftmittel auf keine wie immer geartete Vorschriften bezüglich VEXAT (Verordnung explosionsfähige Atmosphären) geachtet werden muss. Außerdem ist auch dadurch eine Vergällung des Alkohols im Haus des Herstellers dieses Haftmittels entbehrlich, wodurch sich eine entsprechende Verständigung der Zollbehörden erübrigt.

Das Haftmittel gemäß vorliegender Erfindung sowie ein Verfahren zu dessen Herstellung sind im Folgenden anhand von Ausführungsbeispielen näher erläutert, wozu bemerkt wird, dass die angegebenen Gew.-Teile jeweils auf die Gesamtmasse des Haftmittels bezogen sind.

### Beispiel 1:

In einem ersten Schritt werden zur Zubereitung einer ersten Mischung ca. 22 Gew.-Teile des Terpolymers mit 25 Gew.-Teilen Glycerintriacetat und 2-4 Gew.-Teilen Aqua purificata so vermischt, dass eine zähflüssige durchscheinende Masse entsteht.

In einem zweiten Schritt wird die so hergestellte erste Mischung in einen Mischkneter eingeführt und darin wird in die Mischung 4 Gew.-Teile Paraffinum liquidum in etwa 20 Minuten eingerührt.

In einem dritten Verfahrensschritt werden kurz vorgemischte 2-3 Gew.-Teile Silica und 43-44 Gew.-Teile mikrokristallines CMC-Granulat als Gemenge kontinuierlich unter intensivem Rühren der in Schritt zwei erhaltenen Mischung zugesetzt. Nach ca. 20 Minuten Mischzeit wird die Mischdrehrichtung umgekehrt. Nach weiteren 20 Minuten wird das Mischgut umgepumpt und nach weiteren 20 Minuten hat das Mischgut eine Temperatur von ca. 43-45° C erreicht. Danach kann die fertige Haftmittelmischung in Tuben abgefüllt werden.

### Beispiel 2:

In einem ersten Schritt werden zur Zubereitung einer ersten Mischung ca. 20 Gew.-Teile des Terpolymers mit 29 Gew.-Teilen Glycerintriacetat und 2 Gew.-Teilen Aqua purificata so vermischt, dass eine zähflüssige durchscheinende Masse entsteht.

In einem zweiten Schritt wird die so hergestellte erste Mischung in einen Mischkneter eingeführt und darin wird in die Mischung 6 Gew.-Teile Paraffinum liquidum in etwa 20 Minuten eingerührt.

In einem dritten Verfahrensschritt werden kurz vorgemischte 2 Gew.-Teile Silica und 43 Gew.-Teile mikrokristallines CMC-Granulat als Gemenge kontinuierlich unter intensivem Rühren der in Schritt zwei erhaltenen Mischung zugesetzt. Nach ca. 20 Minuten Mischzeit wird die Mischdrehrichtung umgekehrt. Nach weiteren 20 Minuten wird das Mischgut umgepumpt und nach weiteren 20 Minuten hat das Mischgut eine Temperatur von ca. 43-45° C erreicht. Danach kann die fertige Haftmittelmischung in Tuben abgefüllt werden.

### Beispiel 3:

In einem ersten Schritt werden zur Zubereitung einer ersten Mischung ca. 24 Gew.-Teile des Terpolymers mit 27 Gew.-Teilen Glycerintriacetat und 4 Gew.-Teilen Aqua purificata so vermischt, dass eine zähflüssige durchscheinende Masse entsteht.

In einem zweiten Schritt wird die so hergestellte erste Mischung in einen Mischkneter eingeführt und darin wird in die Mischung 2 Gew.-Teile Paraffinum liquidum in etwa 20 Minuten eingerührt.

In einem dritten Verfahrensschritt werden kurz vorgemischte 6 Gew.-Teile Silica, 36 Gew.-Teile mikrokristallines CMC-Granulat und 1 Gew.-Teil Xanthan als Gemenge kontinuierlich unter intensivem Rühren der in Schritt zwei erhaltenen Mischung zugesetzt. Nach ca. 20 Minuten Mischzeit wird die Mischdrehrichtung umgekehrt. Nach weiteren 20 Minuten wird das Mischgut umgepumpt und nach weiteren 20 Minuten hat das Mischgut eine Temperatur von ca. 43-45° C erreicht. Danach kann die fertige Haftmittelmischung in Tuben abgefüllt werden.

## Patentansprüche

1. Haftmittel für Zahnprothesen im Ober- und Unterkieferbereich in Form einer pastösen, leicht verstreichbaren, Cellulosederivate und/oder Hydrokolloide und/oder mikrokristallines CMC-Granulat, Glycerintriacetat, Silica und Aqua purificata enthaltenden Masse, wobei die Masse ein Terpolymer enthält, **dadurch gekennzeichnet, dass** das Terpolymer eine Zusammensetzung an Monomeren von 57 Gew.-% Vinylacetat, 40 Gew.-% Vinyllaurat und 3 Gew.-% Crotonsäure aufweist.

2. Haftmittel nach Anspruch 1, **dadurch gekennzeichnet, dass** die Masse zusätzlich Xanthan enthält.

3. Haftmittel nach Anspruch 1, **dadurch gekennzeichnet, dass** es eine Masse umfasst, die 20 - 24 Gew.-Teile Terpolymer, 22 - 29 Gew.-Teile Glycerintriacetat, 2 - 6 Gew.-Teile Paraffinum liquidum, 32 - 44 Gew.-Teile Cellulosederivate und/oder Hydrokolloide und/oder mikrokristallines CMC-Granulat, 1 - 3 Gew.-Teile Silica und 2 - 4 Gew.-Teile Aqua purificata aufweist.

4. Haftmittel nach Anspruch 3, **dadurch gekennzeichnet, dass** die Masse 22 Gew.-Teile Terpolymer, 25 Gew.-Teile Glycerintriacetat, 3 Gew.-Teile Aqua purificata, 4 Gew. Teile Paraffinum liquidum, 3 Gew.-Teile Silica, 43 Gew.-Teile mikrokristallines CMC-Granulat und/oder Hydrokolloide und/oder Cellulosederivat aufweist.

5. Haftmittel nach den Ansprüchen 1 und 2, **dadurch gekennzeichnet, dass** die Masse 24 Gew.-Teile Terpolymer, 27 Gew.-Teile Glycerintriacetat, 4 Gew.-Teile Aqua purificata, 2 Gew.-Teile Paraffinum liquidum, 6 Gew.-Teile Silica, 36 Gew.-Teile mikrokristallines CMC-Granulat und/oder Hydrokolloide und/oder Cellulosederivat und 1 Gew.-Teil Xanthan aufweist.

6. Verfahren zur Herstellung eines Haftmittels nach einem der Ansprüche 1 bis 5, **dadurch gekennzeichnet, dass** eine erste Mischung durch Vermischen von Terpolymer mit der Monomerzusammensetzung 57 Gew.-% Vinylacetat, 40 Gew.-% Vinyllaurat und 3 Gew.-% Crotonsäure, Glycerintriacetat und Aqua purificata zu einer zähflüssigen durchscheinenden Masse zubereitet wird, dass dann in die so hergestellte Masse in einem Mischkneter Paraffinum liquidum eingerührt wird, dass danach ein vorgemischtes Gemenge von Silica und mikrokristallinem CMC-Granulat kontinuierlich unter intensivem Rühren der Mischung zugesetzt wird, worauf die Mischdrehrichtung umgekehrt wird und dass danach das Mischgut umgepumpt und schließlich auf eine Temperatur von ca. 43-45° C gebracht wird.

7. Verfahren nach Anspruch 6, **dadurch gekennzeichnet, dass** dem vorgemischten Gemenge von Silica und mikrokristallinem CMC-Granulat Xanthan zugesetzt wird.

## Claims

1. Bonding agent for dental prostheses in the upper and lower jaw region in the form of a paste-like, easily spreadable mass containing cellulose derivates and/or hydrocolloids and/or microcrystalline CMC granulate, glyceryl triacetate, silicon dioxide and aqua purificata, wherein the mass contains a terpolymer, **characterised in that** the terpolymer has a composition of monomers of 57% by weight vinyl acetate, 40% by weight vinyl laurate and 3% by weight crotonic acid.

2. Bonding agent according to claim 1, **characterised in that** the mass also contains xanthan.

3. Bonding agent according to claim 1, **characterised in that** it comprises a mass which has 20-24 parts by weight of terpolymer, 22-29 parts by weight of glyceryl triacetate, 2-6 parts by weight of paraffinum liquidum, 32-44 parts by weight of cellulose derivates and/or hydrocolloids and/or microcrystalline CMC granulate, 1-3 parts by weight of silicon dioxide and 2-4 parts by weight of aqua purificata.

4. Bonding agent according to claim 3, **characterised in that** the mass has 22 parts by weight of terpolymer, 25 parts by weight of glyceryl triacetate, 3 parts by weight of aqua purificata, 4 parts by weight of paraffinum liquidum, 3 parts by weight of silicon dioxide, 43 parts by weight of microcrystalline CMC granulate and/or hydrocolloids and/or cellulose derivate.

5. Bonding agent according to claims 1 and 2, **characterised in that** the mass has 24 parts by weight of terpolymer, 27 parts by weight of glyceryl triacetate, 4 parts by weight of aqua purificata, 2 parts by weight of paraffinum liquidum, 6 parts by weight of silicon dioxide, 36 parts by weight of microcrystalline CMC granulate and/or hydrocolloids and/or cellulose derivate and 1 part by weight of xanthan.

6. Method for producing a bonding agent according to one of claims 1 to 5, **characterised in that** a first mixture is prepared by mixing terpolymer with the monomer composition of 57% by weight vinyl acetate, 40% by weight vinyl laurate and 3% by weight crotonic acid, glyceryl triacetate and aqua purificata to form a viscous, translucent mass, paraffinum liquidum is then stirred into the mass produced in this way in a mixing kneader, after that, a pre-mixed blend of silicon dioxide and microcrystalline CMC granulate is continuously added by intensive stirring of the mixture, whereupon the mixing rotational direction is reversed, and, after that, the mix is transfer-pumped and finally brought to a temperature of c. 43-45°C.

7. Method according to claim 6, **characterised in that** xanthan is added to the pre-mixed blend of silicon dioxide and microcrystalline CMC granulate.

## Revendications

1. Produit adhésif pour prothèses dentaires dans la région de la mâchoire supérieure et inférieure sous la forme d'une masse pâteuse, facile à étaler, contenant des dérivés de cellulose et/ou des hydrocolloïdes et/ou des granulés de CMC microcristallins, du triacétate de glycérol, de la silice et de l'aqua purificata, la masse contenant un terpolymère, **caractérisé en ce que** le terpolymère présente une composition en monomères de 57 % en poids d'acétate de vinyle, 40 % en poids de laurate de vinyle et 3 % en poids d'acide crotonique.

2. Produit adhésif selon la revendication 1, **caractérisé en ce que** la masse contient en outre du xanthane.

3. Produit adhésif selon la revendication 1, **caractérisé en ce qu'**il comprend une masse qui présente de 20 à 24 parties en poids de terpolymère, de 22 à 29 parties en poids de triacétate de glycérol, de 2 à 6 parties en poids de paraffinum liquidum, de 32 à 44 parties en poids de dérivés de cellulose et/ou d'hydrocolloïdes et/ou de granulés de CMC microcristallins, de 1 à 3 parties en poids de silice et 2 à 4 parties en poids d'aqua purificata.

4. Produit adhésif selon la revendication 3, **caractérisé en ce que** la masse présente 22 parties en poids de terpolymère, 25 parties en poids de triacétate de glycérol, 3 parties en poids d'aqua purificata, 4 parties en poids de paraffinum liquidum, 3 parties en poids de silice, 43 parties en poids de granulés de CMC microcristallins et/ou d'hydrocolloïdes et/ou de dérivé de cellulose.

5. Produit adhésif selon les revendications 1 et 2, **caractérisé en ce que** la masse présente 24 parties en poids de terpolymère, 27 parties en poids de triacétate de glycérol, 4 parties en poids d'aqua purificata, 2 parties en poids de paraffinum liquidum, 6 parties en poids de silice, 36 parties en poids de granulés de CMC microcristallins et/ou d'hydrocolloïdes et/ou de dérivé de cellulose et 1 partie en poids de xanthane.

6. Procédé de préparation d'un produit adhésif selon l'une des revendications 1 à 5, **caractérisé en ce qu'**un premier mélange est préparé en mélangeant un terpolymère présentant la composition en monomères de 57 % en poids d'acétate de vinyle, 40 % en poids de laurate de vinyle et 3 % en poids d'acide crotonique, de triacétate de glycérol et d'aqua purificata pour obtenir une masse translucide visqueuse, que du paraffinum liquidum est ensuite introduit dans la masse ainsi préparée dans un malaxeur mélangeur, qu'une composition prémélangée de silice et de granulés de CMC microcristallins est ensuite ajoutée au mélange en continu sous agitation intensive, après quoi le sens de rotation de mélange est inversé, et que la matière mélangée est ensuite pompée et finalement portée à une température de 43 à 45 °C environ.

7. Procédé selon la revendication 6, **caractérisé en ce que** du xanthane est ajouté à la composition prémélangée de silice et de granulés de CMC microcristallins.
